(19) Europäisches Patentamt
European Patent Office
Office européen des brevets

(11) **EP 4 763 052 A1**

(12) **EUROPÄISCHE PATENTANMELDUNG**

(43) Veröffentlichungstag:
**24.06.2026   Patentblatt 2026/26**

(21) Anmeldenummer: **24222453.3**

(22) Anmeldetag: **20.12.2024**

(51) Internationale Patentklassifikation (IPC):
**A61B 5/00** (2006.01)    **A61B 90/00** (2016.01)

(52) Gemeinsame Patentklassifikation (CPC):
**A61B 5/0077; A61B 5/6878; A61B 34/20;**
**A61B 90/361;** A61B 5/064; A61B 2034/2055;
A61B 2090/3983; A61B 2505/05; A61B 2576/02

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Medivation AG**
**5400 Baden (CH)**

(72) Erfinder:
• **Stifter, Jan**
**5425 Schneisingen (CH)**

• **Schwägli, Tobias**
**4500 Solothurn (CH)**
• **Imboden, Gregor**
**8045 Zürich (CH)**
• **Schmid, Patricia**
**8051 Zürich (CH)**
• **Moor, Roman**
**5000 Aarau (CH)**

(74) Vertreter: **Herrmann, Johanna**
**Industrial Property Services GmbH**
**Rosenweg 14**
**4303 Kaiseraugst (CH)**

(54) **KNOCHENFIXIERTER OBERFLÄCHENSCANNER**

(57) Ein knochenfixierter Oberflächenscanner zur Erfassung einer Oberfläche umfasst zumindest eine Knochenfixierung (6) zur Befestigung am Knochen (7), ein optisches Erfassungsgerät (1), zumindest eine Quelle für strukturierte Beleuchtung (2), ein Trägerelement (3), auf welchem mindestens das optische Erfassungsgerät (1) und zumindest die Quelle für strukturierte Beleuchtung (2) in einer definierten Position befestigbar sind, sowie ein Verbindungselement (4), welches an der Knochenfixierung (6) befestigbar ist. Die Oberfläche befindet sich in einer fixen Position relativ zur Knochenfixierung des knochenfixierten Oberflächenscanners. Das Trägerelement (3) ist auf dem Verbindungselement (4) befestigbar. Das Verbindungselement (4) weist mindestens einen Freiheitsgrad auf, um das Trägerelement (3), das optische Erfassungsgerät (1) und die Quelle für strukturierte Beleuchtung (2) in definierte Positionen gegenüber der Knochenfixierung zu bewegen.

Fig. 1

**Beschreibung**

Hintergrund

Die Erfindung betrifft einen knochenfixierten Oberflächenscanner zur Erfassung einer Oberfläche eines Knochens sowie ein Verfahren zum Betrieb eines knochenfixierten Oberflächenscanners zur Erfassung der Oberfläche. Der knochenfixierte Oberflächenscanner ist dazu ausgebildet, mittels einer Knochenfixierungsvorrichtung an einem Knochen befestigt zu werden und mittels eines optischen Erfassungsgeräts und einer Quelle für strukturierte Beleuchtung die Oberfläche des Knochens zu erfassen. Der knochenfixierte Oberflächenscanner kann zur Registrierung eines CT-Knochenmodells in einer Operation verwendet werden, wobei ein gescanntes Knochenmodell erzeugt wird. Das gescannte Knochenmodell kann mit einem bestehendem CT-Knochenmodell desselben Knochens registriert werden, um die exakte Position der Knochenfixierungsvorrichtung auf dem Knochen zu berechnen. Auf der exakt lokalisierten Knochenfixierungsvorrichtung können auch andere Werkzeuge montiert werden, beispielsweise ein Werkzeug zur Resektion des Knochens.

Stand der Technik

Ein Oberflächenscanner ist ein Gerät, das mithilfe einer Kamera und einer strukturierten Beleuchtung dreidimensionale Objekte oder Umgebungen präzise vermisst und digitalisiert. Die strukturierte Beleuchtung kann zum Beispiel ein Linienlaser oder ein Streifenprojektor sein. Er wird in verschiedenen Bereichen eingesetzt, darunter Architektur, Industrie, Archäologie und Forensik, um genaue 3D-Modelle oder Karten zu erstellen.

Ein Oberflächenscanner mit einem Linienlaser projiziert eine Linie auf die Oberfläche des zu scannenden Objekts. Eine Kamera erfasst die Verzerrung dieser Linie auf dem Objekt. Aus den ermittelten Daten kann die Form der Oberfläche berechnet werden. Durch das systematische Abtasten des gesamten Objekts entsteht ein vollständiges 3D-Modell. Die genaue Position der Kamera im 3D-Modell kann berechnet werden. Diese Technik ist besonders für die Vermessung komplexer Oberflächen geeignet, da sie eine hohe Präzision und Detailgenauigkeit bietet.

In einem chirurgischen Eingriff, welcher mithilfe von CT-Knochenmodellen geplant wird, wird üblicherweise ein Referenzelement direkt am Knochen fixiert. Das Referenzelement wird von einem externen Navigationssystem getrackt. Um die Position des Referenzelements im Knochen zu kennen, werden gewisse Knochenmarkierungen mit einem speziellen Instrument abgetastet. Ein solches Instrument kann aus einer Spitze, einem Griff und einem zusätzlichen Referenzelement bestehen. Beim Abtasten der Knochenmarkierungen wird sowohl die Position des Instruments als auch die Position des Referenzelements getrackt. Wenn genügend Punkte der Knochenoberfläche aufgezeichnet worden sind, kann man die aufgezeichneten Punkte der Knochenoberfläche mit dem CT-Modell ausrichten. Dieser Prozess wird im Folgenden als Registrierung bezeichnet.

Wenn die Registrierung erfolgreich abgeschlossen ist, können Instrumente zur Bearbeitung des Knochens verwendet werden, welche ein Referenzelement aufweisen. Da die Geometrie der verwendeten Instrumente bekannt ist, kann durch Kamera-Tracking des Referenzelements auf dem Instrument und des knochenfixierten Referenzelements die genaue Position des Instruments in Bezug auf den Knochen in Echtzeit berechnet und auf einem Bildschirm dargestellt werden.

Aufgabe der Erfindung

Aufgabe der Erfindung ist es, die Knochen-Registrierung zu vereinfachen und exakter durchzuführen als mit herkömmlichen Navigationssystemen.

Beschreibung der Erfindung

Die Lösung der Aufgabe erfolgt durch einen knochenfixierten Oberflächenscanner gemäss Anspruch 1. Vorteilhafte Ausführungsbeispiele des knochenfixierten Oberflächenscanners sind Gegenstand der abhängigen Ansprüche.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für den erfindungsgemässen knochenfixierten Oberflächenscanner. Die Beschreibung eines bestimmten knochenfixierten Oberflächenscanners ist nur als beispielhaft anzusehen. In der Beschreibung und den Ansprüchen werden die Begriffe "enthalten", "umfassen", "aufweisen" als "enthalten, aber nicht beschränkt auf" interpretiert.

Ein knochenfixierter Oberflächenscanner zur Erfassung einer Oberfläche umfasst eine Knochenfixierung zur Befestigung an einem Knochen, wobei sich die Oberfläche in einer fixen Position relativ zur Knochenfixierung des knochenfixierten Oberflächenscanners befindet. Ferner umfasst der knochenfixierte Oberflächenscanner zumindest ein optisches Erfassungsgerät, zumindest eine Quelle für strukturierte Beleuchtung, ein Trägerelement, auf welchem das mindestens eine optische Erfassungsgerät und die mindestens eine Quelle für strukturierte Beleuchtung in einer definierten Position befestigbar sind. Ein Verbindungselement ist an der Knochenfixierung befestigbar. Das Trägerelement ist auf dem Verbindungselement befestigbar, wobei das Verbindungselement mindestens einen Freiheitsgrad aufweist, um das Trägerelement, das optische Erfassungsgerät und die Quelle für strukturierte Beleuchtung in definierte Positionen gegenüber der Knochenfixierung zu bewegen.

Nach einem Ausführungsbeispiel stehen eine Längsachse des mindestens einen optischen Erfassungsgeräts und eine Längsachse der mindestens einen Quelle für strukturierte Beleuchtung in einem Winkel von 10 Grad bis einschliesslich 60 Grad zueinander.

Nach einem Ausführungsbeispiel enthält das Verbindungselement mehrere Verbindungsteilelemente.

Nach einem Ausführungsbeispiel ist ein Muster auf die zu scannende Oberfläche auftragbar, wodurch die zu scannende Oberfläche von dem optischen Erfassungsgerät rekonstruierbar ist.

Nach einem Ausführungsbeispiel ist zumindest eine Region auf der Oberfläche des Knochens markierbar, wodurch zumindest eine markierte Region erhältlich ist, wobei die zumindest eine markierte Region mittels des optischen Erfassungsgeräts erkennbar ist.

Nach einem Ausführungsbeispiel ist zumindest eine Lichtquelle in der Nähe des optischen Erfassungsgeräts zur Beleuchtung der zu scannenden Oberfläche befestigbar.

Nach einem Ausführungsbeispiel beinhaltet das Verbindungselement zumindest einen Verbindungselementantrieb, um das Verbindungselement in mindestens einem Freiheitsgrad zu bewegen.

Nach einem Ausführungsbeispiel umgibt ein Gehäuse zumindest das Trägerelement.

Nach einem Ausführungsbeispiel umgibt eine sterile Abdeckung zumindest das Trägerelement.

Ein erfindungsgemässer knochenfixierter Oberflächenscanner umfasst somit eine Knochenfixierung, welche starr an einem Knochen fixiert werden kann. Ferner umfasst der knochenfixierte Oberflächenscanner ein Verbindungselement, welches einen oder mehrere Freiheitsgrade aufweist und an der Knochenfixierung befestigt wird. Ferner umfasst der knochenfixierte Oberflächenscanner ein Trägerelement, welches starr mit dem Verbindungselement gekoppelt ist. Durch den einen oder die mehreren Freiheitsgrade des Verbindungselements kann das Trägerelement in verschiedene definierte Positionen relativ zur Knochenfixierung bewegt werden. Ferner umfasst der knochenfixierte Oberflächen-canner ein oder mehrere optische Erfassungsgeräte, welche starr auf dem Trägerelement befestigt sind. Ferner umfasst der knochenfixierte Oberflächenscanner eine oder mehrere Quellen für strukturierte Beleuchtung, welche ebenfalls starr auf dem Trägerelement befestigt sind. Nach einem Ausführungsbeispiel ist das optische Erfassungsgerät eine Kamera. Die Quelle für strukturierte Beleuchtung kann eine Lichtquelle umfassen. Insbesondere kann die Lichtquelle zur Emission von strukturiertem Licht ausgebildet sein. Unter strukturiertem Licht wird erfindungsgemäss ein Lichtstrahl oder ein Lichtstrahlbündel verstanden, welches auf einer beleuchteten Oberfläche eine Linie oder ein Linienmuster erzeugt. Die Linien des Linienmusters können verschiedene Farben beinhalten. Nach einem Ausführungsbeispiel ist die Quelle für strukturierte Beleuchtung ein Linienlaser.

Nach einem Ausführungsbeispiel beinhaltet das Verbindungselement einen motorisierten Verbindungselementantrieb, welcher das Verbindungselement in den vorhandenen Freiheitsgraden in definierte Positionen gegenüber der Knochenfixierung bewegt. Nach einem Ausführungsbeispiel enthält der knochenfixierte Oberflächenscanner zusätzlich ein Gehäuse, welches das Trägerelement und alle daran befestigten Teile, beispielsweise das optische Erfassungsgerät und die Quelle für strukturierte Beleuchtung, umgibt. Nach einem Ausführungsbeispiel ist die Befestigung des Trägerelements am Verbindungselement so gestaltet, dass das Trägerelement entfernt werden kann und dass auch andere Instrumente, wie zum Beispiel ein Werkzeug zur Resektion zumindest eines Knochens, am Verbindungselement befestigt werden können. Nach einem Ausführungsbeispiel enthält das Trägerelement einen Werkzeughalter für ein Werkzeug zur Knochenresektion. Die zu scannende Oberfläche ist nach einem Ausführungsbeispiel der Knochen, auf welchem der knochenfixierte Oberflächenscanner fixiert ist. Die Knochenoberfläche kann zumindest ein Element bestehend aus einem Knochen oder Knorpel umfassen. Die zu scannende Oberfläche kann auch einen anderen Knochen umfassen, welcher starr mit dem Knochen verbunden ist, auf welchem der knochenfixierte Oberflächenscanner fixiert ist. Alternativ oder zusätzlich können auch andere anatomische Strukturen wie Gewebe, Haut, Weichteile usw. gescant werden. Falls bei der Operation andere Instrumente zur Hilfe genommen werden, welche temporär starr mit dem zu scannenden Knochen verbunden sein können, wie zum Beispiel Knochenpins oder Retraktoren, so können diese anderen Instrumente ebenfalls gescant werden. Der knochenfixierter Oberflächenscanner kann die gescannte Oberfläche anschliessend in Regionen unterteilen, welche die zuvor genannten Strukturen enthalten, also beispielsweise Knochen, Knorpel, Gewebe, Haut, Weichteile, Knochenpins und Retraktoren.

Ein Verfahren zum Betrieb eines knochenfixierten Oberflächenscanners zur Erfassung einer Oberfläche umfassend den Schritt einer Befestigung einer Knochenfixierung des knochenfixierten Oberflächenscanners an einem Knochen, wobei der knochenfixierte Oberflächenscanner zumindest ein optisches Erfassungsgerät, zumindest eine Quelle für strukturierte Beleuchtung, zumindest ein Trägerelement und zumindest ein Verbindungselement umfasst. Auf dem Trägerelement werden das mindestens eine optische Erfassungsgerät und die mindestens eine Quelle für strukturierte Beleuchtung in einer definierten Position befestigt. Ein Verbindungselement wird an der Knochenfixierung befestigt. Das Trägerelement wird auf dem Verbindungselement befestigt. Das Verbindungselement weist mindestens einen Freiheitsgrad auf, um das Trägerelement, das optische Erfassungsgerät und die Quelle für strukturierte Beleuchtung in definierte Positionen gegenüber der Knochenfixierung zu bewegen.

Das Verfahren zum Betrieb eines knochenfixierten Oberflächenscanners zur Erfassung einer Oberfläche umfasst ferner den Schritt des Scannens der Oberfläche und einer anschliessenden Registrierung der Oberfläche mittels des optischen Erfassungsgeräts und ein Ermitteln einer registrierten Position der Knochenfixierung im Knochen.

Das Verfahren zum Betrieb eines knochenfixierten Oberflächenscanners zur Erfassung einer Oberfläche umfasst ferner den Schritt eines Entfernens des Trägerelements, des optischen Erfassungsgeräts und der Quelle für strukturierte Beleuchtung, sowie den Schritt einer anschliessenden Montage eines Werkzeugs zur Resektion eines Knochens.

Das Verfahren zum Betrieb eines knochenfixierten Oberflächenscanners zur Erfassung einer Oberfläche umfasst ferner den Schritt einer Planung eines Werkzeugpfades basierend auf der registrierten Position der Knochenfixierung im Knochen.

Optional kann vor dem Scannen der Oberfläche eine Behandlung der Oberfläche zur Verbesserung des Scans mittels eines Bearbeitungsmittels erfolgen. Das Bearbeitungsmittel kann beispielsweise als Mattierungsspray oder als Pulver ausgebildet sein. Ein Mattierungsspray aus Kreide kann helfen, eine glänzende oder dunkle Oberfläche mit einer dünnen Schicht zu überziehen, damit die Oberfläche weiss, matt und hell wird. Das Pulver kann ebenfalls aufgetragen werden, um eine glänzende oder dunkle Oberfläche mit einer dünnen Schicht zu überziehen, damit die Oberfläche weiss, matt und hell wird. Diese Eigenschaften verbessern die Qualität eines Oberflächenscans. Die dünne Schicht kann beispielsweise als Kreideschicht ausgebildet sein. Die Kreideschicht kann selbstverflüchtigend sein oder manuell entfernt werden. Wenn eine Knochenoberfläche gescannt wird, kann diese zuvor von Knorpel befreit werden, damit die Knochenoberfläche besser sichtbar ist.

Anschliessend kann eine Bearbeitung des Knochens mit einem Werkzeug zur Resektion des Knochens erfolgen. Mit dem Werkzeug zur Resektion eines Knochens kann gegebenenfalls auch ein benachbarter Knochen bearbeitet werden, der starr mit dem Knochen verbunden ist. Nach einem Ausführungsbeispiel kann nach dem Abschluss der Bearbeitung des Knoches das Trägerelement vom Verbindungselement entfernt werden. Nach einem alternativen Ausführungsbeispiel kann das Trägerelement und das Verbindungselement von der Knochenfixierung entfernt werden.

Optional erfolgt ein erneutes Scannen der Oberfläche zur Überprüfung der Knochenfixierung und zur Überprüfung des bearbeiteten Knochens.

Der erfindungsgemässe knochenfixierte Oberflächenscanner ist somit auf einem Knochen fixiert. Mit anderen Worten ist die Knochenfixierung des knochenfixierten Oberflächenscanners starr mit dem Knochen verbunden. Mittels des Verbindungselements kann zumindest das Trägerelement und die daran befestigten Teile des knochenfixierten Oberflächenscanners sodann in vorgegebene Positionen bewegt werden. Mit dem optischen Erfassungsgerät wird ein Teil der Knochenoberfläche erfasst, mittels der Quelle für strukturierte Beleuchtung kann die Rekonstruktion der Oberfläche erfolgen, sodass eine rekonstruierte Oberfläche erhältlich ist. Die rekonstruierte Oberfläche kann anschliessend mit einem CT-Modell des Knochens registriert werden. Die Oberfläche ist insbesondere als eine dreidimensionale Oberfläche ausgebildet. Selbstverständlich kann die Oberfläche auch als eine zweidimensionale Oberfläche ausgebildet sein. Durch die Registrierung ist die exakte Position der Knochenfixierung und des knochenfixierten Oberflächenscanners bekannt.

Ein grosser Vorteil dieser Erfindung ist die direkte Fixierung eines Oberflächenscanners auf dem Knochen. Durch Verwendung des erfindungsgemässen knochenfixierten Oberflächenscanners wird die Registrierung exakter als mit standardmässig verwendeten grossen, externen Navigationssystemen mit knochenfixierten Referenzelementen und handgeführten Pointerinstrumenten. Auf der Knochenfixierung des knochenfixierten Oberflächenscanners können auch andere Instrumente montiert werden, wie zum Beispiel Instrumente zur Resektion zumindest eines Knochens. Ein Tracking dieser anderen Instrumente kann während der Operation wegfallen, da die Position der Knochenfixierung bekannt ist. Demzufolge wird während der Operation kein grosses, externes Navigationssystem benötigt, welches viel Platz einnimmt. Aufgrund seiner Baugrösse kann das externe Navigationssystem eine Seite des Patienten für sich in Anspruch nehmen, da sichergestellt werden muss, dass die Referenzelemente auf den Instrumenten immer im Blickfeld des Navigationssystems sind. Der erfindungsgemässe knochenfixierte Oberflächenscanner weist hingegen eine geringere Anzahl an Bauteilen auf und weist somit nicht nur eine kompaktere Bauweise auf, sondern kann auch kostengünstiger hergestellt werden als grosse, externe Navigationssysteme.

Der Schritt der Registrierung kann nach einem Ausführungsbeispiel auch weggelassen werden, falls kein CT-Modell verfügbar ist. In diesem Arbeitsablauf wird direkt mit der gescannten Oberfläche gearbeitet, das heisst, eine allfällige Knochenresektion wird auf der gescannten Oberfläche geplant und durchgeführt.

Das Gehäuse, welches das Trägerelement und alle daran befestigten Teile umgibt, kann sterilisierbar sein. Nach einem anderen Ausführungsbeispiel ist zumindest das Trägerelement, das optische Erfassungsgerät und die Quelle für strukturierte Beleuchtung von einer sterilen Abdeckung umhüllt, sodass nur die Knochenfixierung des knochenfixierten Oberflächenscanners sterilisierbar sein muss.

Nach einem Ausführungsbeispiel können bestimmte Regionen auf der Knochenoberfläche markiert werden, beispielsweise durch einen Stift, welche mittels des optischen Erfassungsgeräts als markierte Regionen erkennbar sind. Die markierten Regionen können Stellen auf der Knochenoberfläche sein, welche garantiert frei von Knorpel und Gewebe sind. Die markierten Regionen können bei der Registrierung helfen. Es können auch mehrere Regionen mit verschiedenen Farben markiert werden, beispielsweise kann die markierte Region der Knochenoberfläche in blauer Farbe und ein Knorpel oder ein Gewebe in roter Farbe markiert werden, wobei die Verwendung von verschiedenen Farben ebenfalls bei der Registrierung helfen kann.

Nach einem Ausführungsbeispiel kann ein Muster auf der zu scannenden Oberfläche aufgebracht werden. Das Muster kann vom optischen Erfassungsgerät erkannt werden und zur Rekonstruktion der Oberfläche verwendet werden. Das Muster kann beispielsweise durch eine Folie aufgetragen werden, ähnlich wie ein Abziehbild. Beispielsweise befindet sich das Muster auf einer Klebefolie, welche auf den Knochen geklebt werden kann. Das Muster kann beispielsweise durch Befeuchten von der Klebefolie abgelöst werden, sodass das Muster auf der zu scannenden Oberfläche verbleibt.

Nach einem Ausführungsbeispiel kann der knochenfixierte Oberflächenscanner mit dem optischen Erfassungsgerät Instrumente tracken, auf welchen ein Referenzelement fixiert ist. Dadurch kann der knochenfixierte Oberflächenscanner als kleines, kompaktes Navigationssystem eingesetzt werden.

Nach einem Ausführungsbeispiel kann ein Referenzelement am knochenfixierten Oberflächenscanner befestigt werden, sodass der knochenfixierte Oberflächenscanner von einem externen Navigationssystem getrackt werden kann.

Kurzbeschreibung der Zeichnungen

Nachfolgend wird der erfindungsgemässe knochenfixierte Oberflächenscanner anhand eines Ausführungsbeispiels dargestellt. Es zeigen

Fig. 1 eine perspektivische Ansicht des knochenfixierten Oberflächenscanners, wobei das Gehäuse nicht sichtbar ist,

Fig. 2 eine Explosionsdarstellung eines knochenfixierten Oberflächenscanners gemäss eines ersten Ausführungsbeispiels,

Fig. 3 eine perspektivische Ansicht des knochenfixierten Oberflächenscanners, wobei die Knochenfixierung an einem Knochen angebracht ist,

Fig. 4 eine vereinfachte Darstellung des knochenfixierten Oberflächenscanners, wobei die Funktionsweise des Scanners gut erkennbar ist,

Fig. 5 eine vereinfachte Darstellung des knochenfixierten Oberflächenscanners, wobei ein Muster auf den zu scannenden Knochen aufgetragen wurde,

Fig. 6 eine vereinfachte Darstellung des knochenfixierten Oberflächenscanners, wobei bestimmte Regionen auf dem zu scannenden Knochen markiert wurden,

Fig. 7 eine perspektivische Ansicht des knochenfixierten Oberflächenscanners, wobei die Knochenfixierung an einem Knochen angebracht ist,

Fig. 8 eine perspektivische Ansicht des knochenfixierten Oberflächenscanners, wobei zumindest das Trägerelement, welches sich innerhalb vom Gehäuse befindet, von einer sterilen Abdeckung umgeben ist,

Fig. 9 eine perspektivische Ansicht eines knochenfixierten Oberflächenscanners gemäss eines zweiten Ausführungsbeispiels.

Detaillierte Beschreibung der Zeichnungen

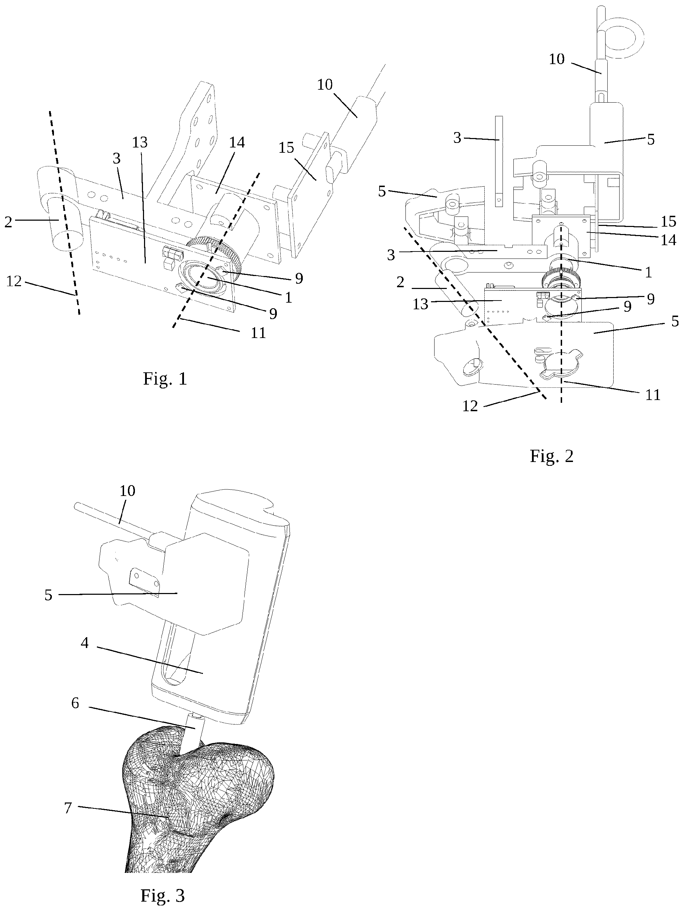

Fig. 1 zeigt eine perspektivische Ansicht des knochenfixierten Oberflächenscanners gemäss eines ersten Ausführungsbeispiels. Ein optisches Erfassungsgerät 1 und eine Quelle für strukturierte Beleuchtung, nach diesem Ausführungsbeispiel ein Linienlaser 2, sind auf einem Trägerelement 3 befestigt. Die Längsachse 11 des optischen Erfassungsgeräts 1 und die Längsachse 12 der Quelle für strukturierte Beleuchtung 2 sind nach diesem Ausführungsbeispiel in einem Winkel von 30° angeordnet. Eine Leiterplatte für die Steuerung der Quelle für strukturierte Beleuchtung 13 weist eine Aussparung für das optische Erfassungsgerät 1 auf. Nach diesem Ausführungsbeispiel sind zwei Lichtquellen 9 auf der Leiterplatte für die Steuerung der Quelle für strukturierte Beleuchtung 13 derart angeordnet, dass das Sichtfeld des optischen Erfassungsgeräts 1 gut beleuchtet ist. Eine Leiterplatte mit Bildsensor 14 ist direkt hinter dem optischen Erfassungsgerät angeordnet, eine weitere Leiterplatte mit Kommunikationsschnittstelle 15 ist ebenfalls in der Nähe angebracht. Die drei Leiterplatten sind elektrisch miteinander verbunden. Ein Kabel 10 führt von der Leiterplatte mit Kommunikationsschnittstelle 15 weg, um Daten zu übertragen.

Fig. 2 zeigt eine Explosionsdarstellung eines knochenfixierten Oberflächenscanners gemäss eines ersten Ausführungsbeispiels. Ein optisches Erfassungsgerät 1 und eine Quelle für strukturierte Beleuchtung, nach diesem Ausführungsbeispiel ein Linienlaser 2, sind auf einem Trägerelement 3 befestigt. Ein Gehäuse 5 umgibt das Trägerelement 3 und die darauf befestigten Komponenten. Die Längsachse des optischen Erfassungsgeräts 11 und die Längsachse 12 der Quelle für strukturierte Beleuchtung sind nach diesem Ausführungsbeispiel in einem Winkel von 30° angeordnet. Eine Leiterplatte für die Steuerung der Quelle für strukturierte Beleuchtung 13 weist eine Aussparung für das optische Erfassungsgerät 1 auf. Nach diesem Ausführungsbeispiel sind zwei Lichtquellen 9 auf der Leiterplatte für die Steuerung der Quelle für strukturierte Beleuchtung 13 derart angeordnet, dass das Sichtfeld des optischen Erfassungsgeräts 1 gut beleuchtet ist. Eine Leiterplatte mit Bildsensor 14 ist direkt hinter dem optischen Erfassungsgerät angeordnet, eine weitere Leiterplatte mit Kommunikationsschnittstelle 15 ist ebenfalls in der Nähe angebracht. Die drei Leiterplatten sind elektrisch miteinander verbunden. Ein Kabel 10 führt von der Leiterplatte mit Kommunikationsschnittstelle 15 weg, um Daten zu übertragen.

Fig. 3 zeigt eine perspektivische Ansicht des knochenfixierten Oberflächenscanners, wobei die Knochenfixierung 6 an einem Knochen 7 angebracht ist. Ein Verbindungselement 4 verbindet das Trägerelement 3 mit der Knochenfixierung 6. Das Verbindungselement 4 weist mehrere Freiheitsgrade auf, welche Bewegungen zulassen, damit eine möglichst grosse Teilfläche der Oberfläche von dem knochenfixierten Oberflächenscanner erfasst werden kann. Bei der Oberfläche handelt es sich insbesondere um eine Knochenoberfläche, eine Gewebeoberfläche oder eine Knorpeloberfläche. Ein Kabel 10 führt aus dem Gehäuse 5 weg, um Daten zu übertragen.

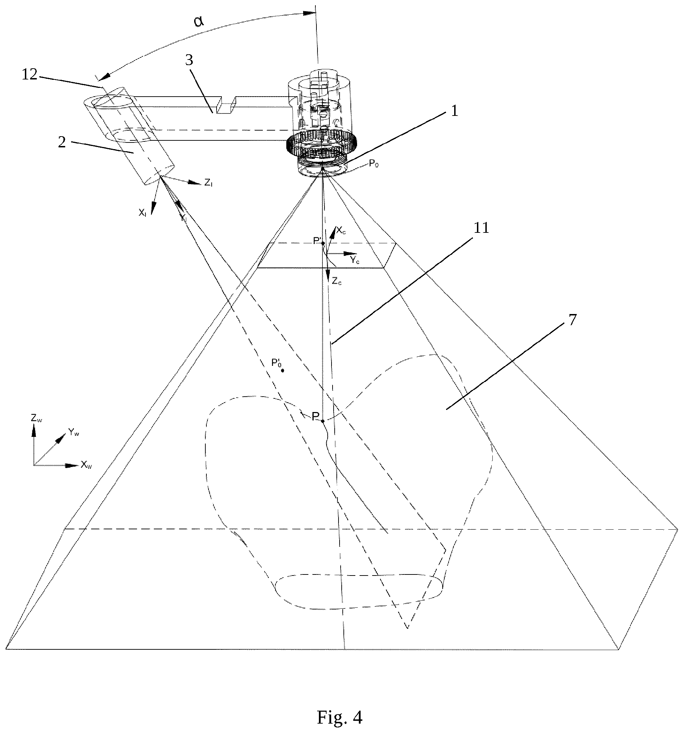

Fig. 4 zeigt eine vereinfachte Darstellung des knochenfixierten Oberflächenscanners, um die Funktionsweise des Oberflächenscanners zu beschreiben. Das optische Erfassungsgerät 1 und die Quelle für strukturierte Beleuchtung 2 stehen in einer definierten (bekannten) Position zueinander. Das von der Quelle für strukturierte Beleuchtung emittierte Licht, welches auf der zu scannenden Oberfläche auftrifft, muss dabei im Blickfeld des optischen Erfassungsgeräts 1 sein. Aus diesem Grund sind das optische Erfassungsgerät 1 und die Quelle für strukturierte Beleuchtung 2 in einem Abstand zueinander angeordnet. Wenn ein Linienlaser als Quelle für die strukturierte Beleuchtung verwendet wird, ist es sinnvoll, dass die Längsachse 11 des optischen Erfassungsgeräts und die Längsachse 12 des Linienlasers in einem Winkel alpha $\alpha$ zueinander stehen. Dieser Winkel alpha liegt typischerweise im Bereich von 10° bis einschliesslich 60°. Die Distanz vom optischen Erfassungsgerät 1 zur Oberfläche, welche gescannt wird, liegt typischerweise im Bereich von 50 mm bis einschliesslich 250 mm. Der Abstand zwischen dem optischen Erfassungsgerät 1 und der Quelle für strukturierte Beleuchtung 2 ergibt sich aus dem Winkel zwischen der Längsachse 11 des optischen Erfassungsgeräts und der Längsachse 12 der Quelle für strukturierte Beleuchtung und der Distanz zwischen dem optischen Erfassungsgerät 1 zur Oberfläche, welche gescannt wird.

Sei $\mathbf{P} = [X, Y, Z]$ ein Punkt im Weltkoordinatensystem und $\mathbf{P'} = [x, y]$ die dazugehörige Projektion in der Bildebene. Die Bildebene ist in Fig. 4 die vor der Kamera befindliche Ebene, welche die Koordinaten $X_c$ und $Y_c$ des Koordina-tensystems $X_c, Y_c, Z_c$ enthält. Durch die Gleichungen (Eq. 1) und (Eq. 2) kann man den 3D Punkt $\mathbf{P}$ durch die Projektion berechnen. Gleichung Eq. 1 beschreibt die projizierte Linie von Punkt $\mathbf{P}$ durch die Kamera, Gleichung Eq. 2 beschreibt die Beleuchtungsebene. Mit der Beleuchtungsebene ist die Ebene gemeint, welche die Koordinaten $X_l$ und $Y_l$ des Koordi-natensystems $X_l, Y_l, Z_l$ enthält. Ihre Intersektion ergibt den gesuchten Punkt $\mathbf{P}$. Der Punkt P liegt somit in der Beleuchtungsebene, die Projektion P' in der Bildebene. Wird das Trägerelement 3 mit dem optischen Erfassungsgerät 1 und der Quelle für strukturierte Beleuchtung bewegt, kann eine 3D Punktwolke erstellt werden, welche jeden Punkt beinhaltet, welcher im Sichtbereich des optischen Erfassungsgeräts 1 und der Quelle für strukturierte Beleuchtung 2 war.

$$\text{Eq. 1:} \qquad \mathbf{P} = \lambda \mathbf{v} + \mathbf{P_0}$$

$$\text{Eq. 2:} \qquad \mathbf{n}^T (\mathbf{P} - \mathbf{P_0'}) = [a\ b\ c]\ [P_X\ P_Y\ P_Z]^T + d = 0$$

wobei $\mathbf{P_0}$ das optische Zentrum der Kamera ist, $\mathbf{v}$ der Einheitsvektor zwischen $\mathbf{P_0}$ und der Projektion vom Punkt $\mathbf{P}$ in der Beleuchtungsebene, $\mathbf{n} = [a, b, c]^T$ der Normalvektor der Beleuchtungsebene und $\mathbf{P_0'}$ ein beliebiger Punkt auf der Beleuchtungsebene ist.

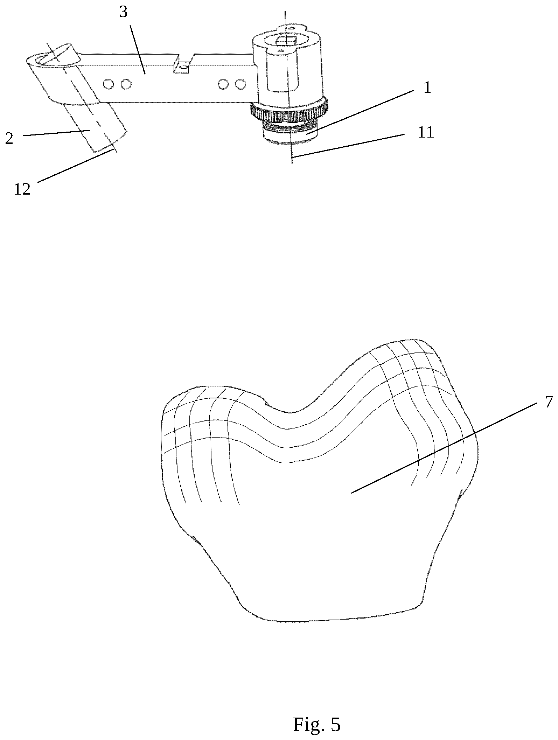

Fig. 5 zeigt eine vereinfachte Darstellung des knochenfixierten Oberflächenscanners, wobei nur das optische Erfassungsgerät 1, die Quelle für strukturierte Beleuchtung 2 und das Trägerelement 3 abgebildet sind. Zusätzlich ist ein Knochen 7 abgebildet, wobei ein bekanntes Muster auf den zu scannenden Knochen 7 aufgetragen wurde. Das Muster kann vom optischen Erfassungsgerät 1 erkannt werden und zur Rekonstruktion der Oberfläche verwendet werden. Das Muster kann beispielsweise durch eine Folie aufgetragen werden.

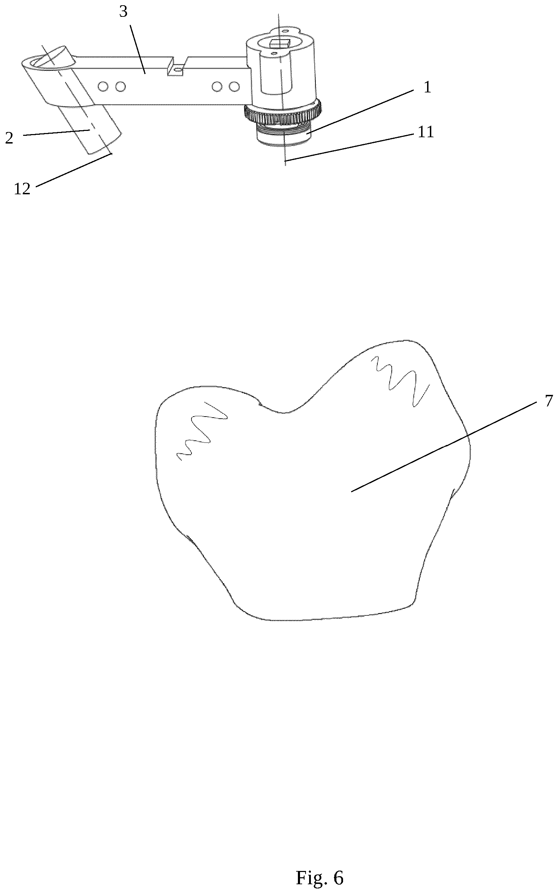

Fig. 6 zeigt eine vereinfachte Darstellung des knochenfixierten Oberflächenscanners, wobei nur das optische Erfassungsgerät 1, die Quelle für strukturierte Beleuchtung 2 und das Trägerelement 3 abgebildet sind. Zusätzlich ist ein Knochen 7 abgebildet, wobei zumindest eine Region auf der Oberfläche des zu scannenden Knochens 7 markiert wurde. Die markierte Region kann vom optischen Erfassungsgerät 1 erkannt werden und zur Rekonstruktion der Oberfläche verwendet werden. Es können beispielsweise Regionen markiert werden, an welchen die Knochenoberfläche sichtbar ist, oder Regionen an welchen die Knorpeloberfläche sichtbar ist oder Regionen, an welchen die Gewebeoberfläche sichtbar ist. An der Gewebeoberfläche wird der Knochen von Gewebe verdeckt. Es können auch Regionen in unterschiedlichen Farben markiert werden, beispielsweise Regionen der Knochenoberfläche in blauer Farbe und Regionen der Knorpelfläche in roter Farbe.

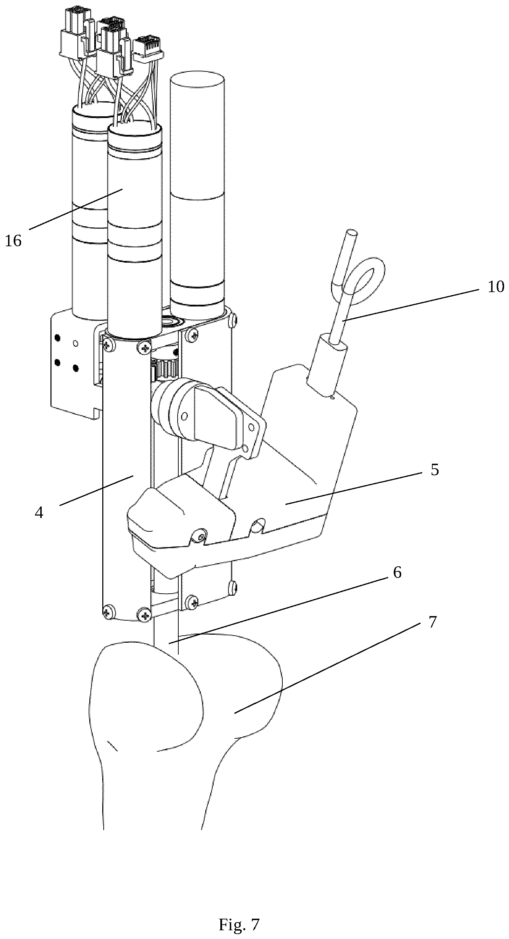

Fig. 7 zeigt eine perspektivische Ansicht des knochenfixierten Oberflächenscanners, wobei die Knochenfixierung 6 an einem Knochen 7 angebracht ist. Das Trägerelement 3 und die daran befestigten Teile werden von einem Gehäuse 5 umgeben. Ein Kabel 10 führt vom Gehäuse 5 weg. Das Verbindungselement 4 ist mit mehreren Freiheitsgraden ausgebildet, was es dem knochenfixierten Oberflächenscanner erlaubt, eine möglichst grosse Teilfläche der Oberfläche des Knochens 7 mit dem optischen Erfassungsgerät 1 zu erfassen, um einen möglichst grossen Teil der Knochenoberfläche zu rekonstruieren. Zusätzlich ist zumindest ein Verbindungselementantrieb 16 vorhanden, welcher das Trägerelement 3 und die daran befestigten Bauteile automatisch in dem oder den vorhandenen Freiheitsgraden bewegen kann. Insbesondere kann der zumindest eine Verbindungselementantrieb als motorisierter Verbindungselementantrieb ausgebildet sein.

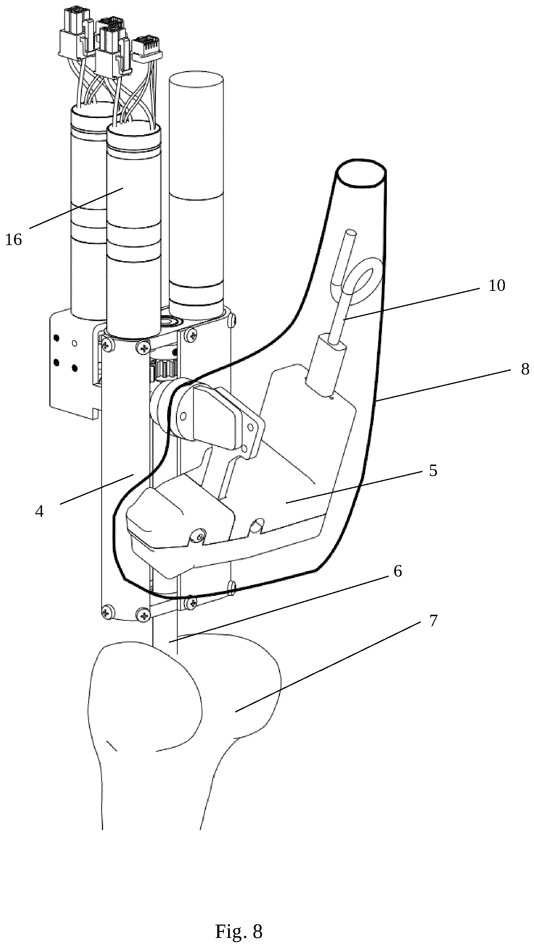

Fig. 8 zeigt eine perspektivische Ansicht des knochenfixierten Oberflächenscanners, wobei die Knochenfixierung 6 an einem Knochen 7 angebracht ist. Das Trägerelement 3 und die daran befestigten Teile werden von einem Gehäuse 5 umgeben. Ein Kabel 10 führt vom Gehäuse 5 weg. Das Verbindungselement 4 ist mit mehreren Freiheitsgraden ausgebildet, was es dem knochenfixierten Oberflächenscanner erlaubt, eine möglichst grosse Teilfläche der Oberfläche des Knochens 7 mit dem optischen Erfassungsgerät 1 zu erfassen, um einen möglichst grossen Teil der Oberfläche des Knochens zu rekonstruieren. Zusätzlich sind Verbindungselementantriebe 16 vorhanden, welche das Trägerelement 3 und die daran befestigten Bauteile automatisch in den vorhandenen Freiheitsgraden bewegen können. Eine sterile Abdeckung 8 umgibt zumindest das Trägerelement 3 und die daran befestigten Bauteile, sowie nach diesem Ausführungsbeispiel zusätzlich das Gehäuse 5.

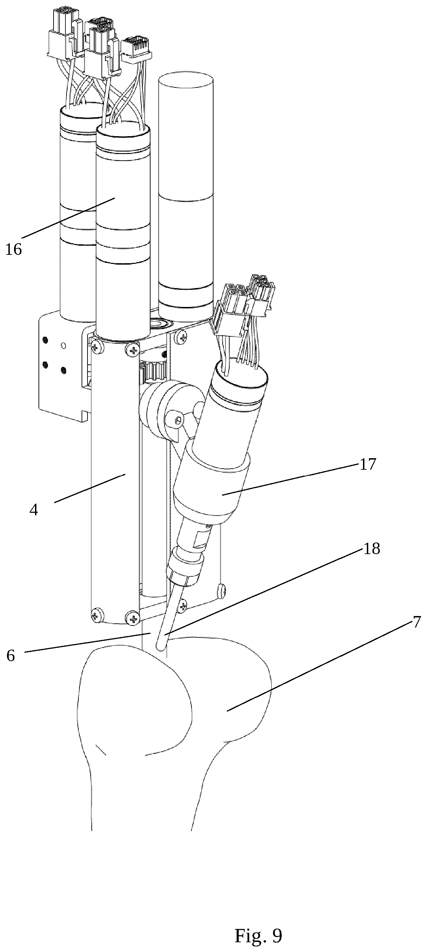

Fig. 9 zeigt eine perspektivische Ansicht eines knochenfixierten Oberflächenscanners gemäss eines zweiten Ausführungsbeispiels, wobei das Trägerelement 3 einen Werkzeughalter 17 für ein Werkzeug 18 zur Knochenresektion enthält. Das Trägerelement 3 kann einfach vom Verbindungselement 4 entfernt werden.

Für eine Fachperson ist offensichtlich, dass viele weitere Varianten zusätzlich zu den beschriebenen Verfahren oder Vorrichtungen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C bis N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

**Patentansprüche**

1. Ein knochenfixierter Oberflächenscanner zur Erfassung einer Oberfläche, umfassend eine Knochenfixierung (6) zur Befestigung an einem Knochen (7), wobei sich die Oberfläche in einer fixen Position relativ zur Knochenfixierung (6) des knochenfixierten Oberflächenscanners befindet, umfassend ferner:

   - zumindest ein optisches Erfassungsgerät (1),
   - zumindest eine Quelle für strukturierte Beleuchtung (2),

- ein Trägerelement (3), auf welchem das mindestens eine optische Erfassungsgerät (1) und die mindestens eine Quelle für strukturierte Beleuchtung (2) in einer definierten Position befestigbar sind,
- ein Verbindungselement (4), welches an der Knochenfixierung (6) befestigbar ist, wobei das Trägerelement (3) auf dem Verbindungselement (4) befestigbar ist, wobei das Verbindungselement (4) mindestens einen Freiheitsgrad aufweist, um das Trägerelement (3), das optische Erfassungsgerät (1) und die Quelle für strukturierte Beleuchtung (2) in definierte Positionen gegenüber der Knochenfixierung zu bewegen.

2. Der knochenfixierte Oberflächenscanner nach Anspruch 1, wobei eine Längsachse (11) des mindestens einen optischen Erfassungsgeräts (1) und eine Längsachse (12) der mindestens einen Quelle für strukturierte Beleuchtung (2) in einem Winkel von 10 Grad bis einschliesslich 60 Grad zueinander stehen.

3. Der knochenfixierte Oberflächenscanner nach einem der Ansprüche 1 oder 2, wobei das Verbindungselement (4) mehrere Verbindungsteilelemente enthält.

4. Der knochenfixierte Oberflächenscanner nach einem der vorhergehenden Ansprüche, wobei ein Muster auf die zu scannende Oberfläche auftragbar ist, wodurch die zu scannende Oberfläche von dem optischen Erfassungsgerät (1) rekonstruierbar ist.

5. Der knochenfixierte Oberflächenscanner nach einem der vorhergehenden Ansprüche, wobei zumindest eine Region auf der Oberfläche des Knochens (7) markierbar ist, wodurch zumindest eine markierte Region erhältlich ist, wobei die zumindest eine markierte Region mittels des optischen Erfassungsgeräts (1) erkennbar ist.

6. Der knochenfixierte Oberflächenscanner nach einem der vorhergehenden Ansprüche, wobei zumindest eine Lichtquelle (9) in der Nähe des optischen Erfassungsgeräts (1) zur Beleuchtung der zu scannenden Oberfläche befestigbar ist.

7. Der knochenfixierte Oberflächenscanner nach einem der vorhergehenden Ansprüche, wobei das Verbindungselement (4) zumindest einen Verbindungselementantrieb (16) beinhaltet, um das Verbindungselement (4) in mindestens einem Freiheitsgrad zu bewegen.

8. Der knochenfixierte Oberflächenscanner nach einem der vorhergehenden Ansprüche, wobei ein Gehäuse (5) zumindest das Trägerelement (3) umgibt.

9. Der knochenfixierte Oberflächenscanner nach einem der vorhergehenden Ansprüche, wobei eine sterile Abdeckung (8) zumindest das Trägerelement (3) umgibt.

10. Der knochenfixierte Oberflächenscanner nach einem der vorhergehenden Ansprüche, wobei das Trägerelement (3) einen Werkzeughalter (17) für ein Werkzeug (18) zur Knochenresektion enthält.

11. Verfahren zum Betrieb eines knochenfixierten Oberflächenscanners zur Erfassung einer Oberfläche umfassend die nachfolgenden Schritte:

a) Befestigen einer Knochenfixierung (6) des knochenfixierten Oberflächenscanners an einem Knochen (7),

a1) wobei der knochenfixierte Oberflächenscanner zumindest ein optisches Erfassungsgerät (1), zumindest eine Quelle für strukturierte Beleuchtung (2), zumindest ein Trägerelement (3) und zumindest ein Verbindungselement (4) umfasst,
a2) wobei auf dem Trägerelement (3) das mindestens eine optische Erfassungsgerät (1) und die mindestens eine Quelle für strukturierte Beleuchtung (2) in einer definierten Position befestigt werden,
a3) wobei ein Verbindungselement (4) an der Knochenfixierung (6) befestigt wird,
a4) wobei das Trägerelement (3) auf dem Verbindungselement (4) befestigt wird,
a5) wobei das Verbindungselement (4) mindestens einen Freiheitsgrad aufweist, um das Trägerelement (3), das optische Erfassungsgerät (1) und die Quelle für strukturierte Beleuchtung (2) in definierte Positionen gegenüber der Knochenfixierung zu bewegen,

b) Scannen der Oberfläche und anschliessend Registrierung der Oberfläche mittels des optischen Erfassungsgeräts (1) und Ermitteln einer registrierten Position der Knochenfixierung im Knochen,
c) Entfernen des Trägerelements (3), des optischen Erfassungsgeräts (1) und der Quelle für strukturierte Beleuchtung (2),
d) Montage eines Werkzeugs zur Resektion eines Knochens,
e) Planung eines Werkzeugpfades basierend auf der registrierten Position der Knochenfixierung im Knochen.

12. Verfahren nach Anspruch 11, wobei vor dem Scannen der Oberfläche eine Bearbeitung der Oberfläche durch ein Bearbeitungsmittel erfolgt.

Fig. 1

Fig. 2

Fig. 3

Fig. 4

3

2

12

1

11

7

Fig. 5

Fig. 6

Fig. 7

Fig. 8

Fig. 9

Europäisches
Patentamt
European
Patent Office
Office européen
des brevets

## EUROPÄISCHER RECHERCHENBERICHT

Nummer der Anmeldung

EP 24 22 2453

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2021/030481 A1 (WALTER WILLIAM L [AU] ET AL) 4. Februar 2021 (2021-02-04)<br>* Abbildungen 2,3 *<br>* Absätze [0069] - [0072], [0090] *<br>----- | 1-9,11,<br>12<br>10 | INV.<br>A61B5/00<br>A61B90/00 |
| A | US 2020/030038 A1 (WANG HAO [CA] ET AL) 30. Januar 2020 (2020-01-30)<br>* Abbildungen 22A-B,29 *<br>* Absätze [0157] - [0159], [0184] *<br>----- | 2,7,8 | |
| A | US 2022/331016 A1 (HLADIO ANDRE NOVOMIR [CA] ET AL) 20. Oktober 2022 (2022-10-20)<br>* Abbildung 9E *<br>* Absatz [0170] *<br>----- | 9 | |
| Y | US 2011/190637 A1 (KNOBEL BRUNO [CH] ET AL) 4. August 2011 (2011-08-04)<br>* Abbildung 15 *<br>* Absätze [0152] - [0154] *<br>----- | 10 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| Den Haag | 9. April 2025 | Oancea, A |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**  EP 24 22 2453

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-04-2025

| Im Recherchenbericht angeführtes Patentdokument | Datum der Veröffentlichung | Mitglied(er) der Patentfamilie | | Datum der Veröffentlichung |
|---|---|---|---|---|
| US 2021030481 A1 | 04-02-2021 | AU | 2018321607 A1 | 05-03-2020 |
| | | CA | 3073335 A1 | 28-02-2019 |
| | | EP | 3672513 A1 | 01-07-2020 |
| | | JP | 2020531223 A | 05-11-2020 |
| | | US | 2021030481 A1 | 04-02-2021 |
| | | WO | 2019036752 A1 | 28-02-2019 |
| US 2020030038 A1 | 30-01-2020 | AU | 2016212656 A1 | 03-08-2017 |
| | | CA | 2973606 A1 | 04-08-2016 |
| | | CN | 107847278 A | 27-03-2018 |
| | | EP | 3250143 A1 | 06-12-2017 |
| | | US | 2016166333 A1 | 16-06-2016 |
| | | US | 2020030038 A1 | 30-01-2020 |
| | | WO | 2016119053 A1 | 04-08-2016 |
| US 2022331016 A1 | 20-10-2022 | US | 2019142524 A1 | 16-05-2019 |
| | | US | 2022331016 A1 | 20-10-2022 |
| | | US | 2023285086 A1 | 14-09-2023 |
| | | WO | 2017185170 A1 | 02-11-2017 |
| US 2011190637 A1 | 04-08-2011 | EP | 2326276 A1 | 01-06-2011 |
| | | US | 2011190637 A1 | 04-08-2011 |
| | | WO | 2010020397 A1 | 25-02-2010 |

EPO FORM P0461

Für nähere Einzelheiten zu diesem Anhang : siehe Amtsblatt des Europäischen Patentamts, Nr.12/82